# EUROPEAN PATENT APPLICATION

(11) **EP 0 535 723 A1**
(43) Date of publication of application: **07.04.1993**
(21) Application number: 92202423.7
(22) Date of filing: 04.08.1992
(51) Int. Cl.: A61K 37/50, C12N 9/96, C12N 9/02

(54) **Buffered formulation of capitals**

(30) Priority: 05.08.1991 US 740255
(71) Applicant: STERLING WINTHROP INC., New York, NY 10016 (US)
(72) Inventor: Na, George C., c/o Sterling Winthrop Inc., New York, New York 10016 (US); Rajagopalan Natarajan, c/o Sterling Winthrop Inc., New York, New York 10016 (US); Revello, Kim Marie, c/o Sterling Winthrop Inc., New York, New York 10016 (US); Yuan, Barbara O-Ching, c/o Sterling Winthrop Inc., New York, New York 10016 (US)
(74) Representative: Le Guen, Gérard

(57) **Abstract**

An aqueous formulation for a conjugate of a mammalian superoxide dismutase with a polyethylene glycol (PEG-SOD), comprising PEG-SOD and a non-chelating buffer in the range from pH 5.0 to pH 6.5. Also disclosed is a method for using the formulation for the prevention and treatment of oxidant injury in a mammal.

## Description

### Background of the Invention

### Field of the Invention

The invention relates to an aqueous formulation for a conjugate of mammalian superoxide dismutase with polyethylene glycol (PEG-SOD) comprising PEG-SOD and non-chelating buffer in the range from pH 5.0 to pH 6.5. The invention further relates to a method of using the formulation for the prevention and treatment of oxidant injury in a mammal.

### Information Disclosure

There are several references that disclose the short-term stability of solutions of unconjugated mammalian super-oxide dismutase (SOD): Johansen US Patents 4,341,867 and 4,390,628 describe solutions of bovine SOD in 10 mM sodium phosphate buffer at pH 7.5 and sodium acetate buffer at pH 4.8. Yasuda US Patent 4,346,174 describes a solution of bovine SOD in 2.5 x 10⁻³ M to 7.5 x 10⁻² M potassium phosphate buffer (pH 7.4) Sagai et al US Patent 4,818,698 discloses recombinant human SOD in 0.1 molar phosphate buffer, pH 7.0, containing 50 millimolar saccharose and refers to its stability at room temperature for one week. Rotilio et al., Biochemistry, 11, p. 2182-2187 (1972) examined the role of copper and zinc in protein conformation and enzyme activity of bovine SOD. They report that "The typical EPR signal of copper of the native bovine enzyme is maintained through the full range of pH 3 to 10." Roe et al., Biochemistry, 27, p. 950-958 (1988) describe the differential scanning calorimetry of bovine copper-zinc SOD in sodium acetate buffer at pH 5.5. Forman and Fridovich, J. Biol. Chem. 248, p. 2645-2649 (1973) report on the effects of metals on the stability of bovine superoxide dismutase. In a paragraph entitled "Effects of pH and Organic Solvents", they state that "superoxide dismutase was stable at pH 11.4 for 24 hours at 24° in a sodium carbonate buffer.

Hamaguchi et al. European Application 225,130 discloses an aqueous solution of 0.067 M phosphate-buffered saline (pH 7.2, 287 mOsm) containing 25 mg of manganese SOD conjugated to polyethylene glycol of molecular weight 5,000 in 5 mL of distilled water. Hamaguchi states "In this manner a ... liposome composition having SOD-PEG (5000) entrapped in stable condition was obtained." This is the only reference of which the inventors are aware that discloses the stability of any solution of a superoxide dismutase that is conjugated to a polyethylene glycol. It differs from the solutions of the invention in that the SOD is not a mammalian Cu/Zn-SOD, but rather is a Mn-SOD from a microorganism. Further, the PEG is directly attached to the SOD by an amide bond, as contrasted with the PEG-SOD of the invention in which the PEG and the SOD are linked through succinyl amide/ester bonds.

Prior to our discovery, no information regarding the stability of the conjugate of a mammalian SOD and a polyethylene glycol was available in the literature. A conjugate of a mammalian SOD and polyethylene glycol or a lower-alkoxypolyethylene glycol connected by a succinyl residue having an ester linkage to the PEG and an amide linkage to an amino function in the SOD will hereinafter be referred to as PEG-S-mSOD. The term polyethylene glycol or PEG when used herein refers to the compound or mixture of compounds of formula R-O-(CH₂CH₂O)ₙOH wherein R is hydrogen or alkyl of one to four carbons and n is an integer from 80 to 25,000. The PEG-S-mSOD solutions, which we obtained from Enzon (South Plainfield, NJ, USA), were formulated by Enzon at pH 7.3 in phosphate buffer. We have now discovered that when aqueous PEG-S-mSOD solutions are prepared in non-chelating buffers at pHs between 5.0 and 6.5, i.e. slightly acidic, they are up to 7-fold more stable than the slightly basic solution of the art (Enzon)

The importance of the long-term stability of aqueous formulations of PEG-S-mSOD arises from the particular utility of PEG-S-mSOD for the treatment and prevention of oxidative injury in a clinical setting. For such treatment, parenteral administration is preferred. A clinically useful parenteral formulation must have a reasonable shelf life under convenient storage conditions or the parenteral solution must be freshly prepared shortly before each use. Obviously, the former is much preferred. Our invention provides an aqueous solution, suitable for parenteral administration that, for the first time, allows storage under convenient conditions for economically practical lengths of time.

### Summary of the Invention

In a composition of matter aspect the invention relates to an aqueous formulation of PEG-S-mSOD comprising PEG-S-mSOD and a non-chelating buffer in the range from pH 5.0 to 6.5, preferably from pH 5.8 to pH 6.2.

In a method aspect, the invention relates to a method of treating or preventing oxidative injury in a mammal which treatment comprises administering to said mammal a therapeutically effective amount of a formulation of PEG-S-mSOD in a non-chelating buffer at pH 5.0 to 6.5.

### Description of the Drawings

Fig. 1 shows the stability of PEG-S-mSOD (1) as the percent of initial enzymic activity as a function of pH, and (2) as mg/mL of free PEG generated as a function of pH, both upon storage at 37°C for 68 days.

Fig. 2 shows the rate of production of free PEG and methoxy PEG in mg/mL (degradation) as a function of time in days for a solution of the invention at pH 6.2 and a solution of the prior art at pH 7.3.

### Description Inclusive of Preferred Embodiments

Superoxide dismutase is the name given to a class of enzymes that catalyze the breakdown of the superoxide anion radical (O₂⁻^{.}) to oxygen and hydrogen peroxide.

SOD is known under the systematic nomenclature of the International Union of Biochemistry as superoxide oxidoreductase and has a classification number of 1.15.1.1. Such substances have been called orgoteins and hemocupreins as well as superoxide dismutases and range in molecular weight from about 400 to about 48,000. The copper-zinc dismutases are a remarkably conserved family with respect to gross structural properties. Without exception, the purified enzymes have been shown to be dimers (molecular weight usually 31,000-33,000) containing two moles each of copper and zinc ions per mole. The enzymes of the manganese/iron family are not as uniform in such basic properties as molecular weight, subunit structure and metal content. Some are dimers; others are tetramers. The content of metal ranges from about 0.5 to 1 mole per mole of subunit polypeptide chain. Naturally occurring Zn/Cu-containing enzymes from mammals and their functionally competent analogs and muteins are considered to be mammalian Zn/Cu superoxide dismutases (mSOD) for the purposes of this invention.

In formulations of the invention mSOD may be of any origin. It is commercially obtained from bovine erythrocytes and human erythrocytes as well as by recombinant synthesis in microorganisms such as E. coli and yeast. Human and bovine SOD are preferred.

For the purposes of the invention mSOD is covalently linked to polymers to provide conjugates that retain enzymic activity. Such conjugates display greatly extended half-lives in the blood stream. Preferred conjugates comprise recombinant or native human SOD or bovine SOD covalently linked to polyethylene glycol (PEG) through a succinyl residue as described in US Patent 4,179,337, which is incorporated herein by reference. The preferred polyethylene glycol has a formula of R-O-(CH₂-CH₂O)ₙOH wherein R is hydrogen or methyl and n is about 112. The preferred PEG is of molecular weight from 1 x 10³ to 2 x 10⁵, preferably 1 x 10³ to 2 x 10⁴, and the conjugate is modified from about 25 to about 99 percent in terms of the available lysine residues, preferably about 45 to about 70 percent. It is preferred that the PEG-S-mSOD have a specific activity greater than about 1000 U/mg of mSOD as determined by the method of Fridovich and McCord [J. Biol. Chem. 244, 6049-6055 (1969)].

The buffers that comprise the other critical component of the formulations of the invention include pharmaceutically acceptable, non-chelating buffers that maintain the pH at 5.0 to 6.5. By pharmaceutically acceptable is meant buffers that are relatively innocuous to the animal organism in medicinal doses of the buffers so that the beneficial properties inherent in the PEG-S-mSOD are not vitiated by side effects ascribable to the buffers. In practicing the present invention it is convenient to use phosphate, adipate, maleate or glutarate buffers. However, other appropriate medicinally acceptable buffers within the scope of the invention include those derived from other mineral acids and organic acids, preferably dicarboxylic organic acids. Phosphate and adipate buffers are particularly preferred. By non-chelating it is meant that the buffer does not contain a component, usually the anionic component, that chelates or sequesters either copper or zinc ions in aqueous solutions.

The buffers may be present in any concentration that is high enough to maintain the pH at a set level but low enough so as not to introduce adverse pharmacological effects from the buffer itself. The minimum concentration of buffer necessary to maintain a set pH is a function of the amount and form of the PEG-S-mSOD in the solution and the relation of the pKₐ of the conjugate acid in the buffer to the desired pH of the solution. More buffer will be required at minimum to establish and maintain the desired pH when the pH is farther from the pKₐ of the buffer or when the concentration of PEG-S-mSOD is higher. Concentrations of PEG-S-mSOD may range from 100 U to 150,000 U/mL. For the preferred compositions of the invention, which contain 8 to 10 mg/mL (20,000 to 30,000 U/mL) of PEG-S-mSOD, the minimum buffer concentration is about 30 mM for phosphate buffer at pH 6.2. Other operable solutions of the invention may have buffer concentrations ranging from about 10 mM to about 500 mM, preferably 30 mM to 50 mM.

Although the invention comprises a solution of PEG-S-mSOD and a buffer, it will be apparent to those in the art that additional components may be added to the composition to secure other advantages for specific uses. For example, for intravenous or intraarterial administration a formulation of the invention may contain, in addition to PEG-S-mSOD and a buffer, isotonic agents, preferably 140 to 150 mM sodium chloride, and, optionally, preservatives.

The mSOD used in the experiments was SOD from bovine liver, purchased from DDI Pharmaceuticals (Mountain View, CA). PEG-S-mSOD was purchased from Enzon Inc. (South Plainfield, NJ) and was equivalent to 3000 ± 600 IU/mg.

Representative compositions of the invention have been prepared as follows:

### Example 1

### Isotonic Phosphate Buffer pH 6.2

A solution of 5.071 g of USP/NF monobasic sodium phosphate monohydrate, 3.552 g of USP/NF dibasic sodium phosphate heptahydrate and 8.500 g of USP/NF sodium chloride in 800 mL of USP/NF water for injection was prepared and brought to 1.000 L with water for injection. The pH was checked and if found to fall outside the range of 6.1 to 6.3, the solution was discarded.

### Example 2

### Isotonic Adipate Buffer pH 5.8

A solution of 5.840 g of adipic acid, 8.500 g of sodium chloride and sufficient sodium hydroxide to achieve a pH of 5.8 in 800 mL of water for injection was prepared and brought to 1.000 L with water for injection.

### Example 3

### Isotonic Phosphate Buffer pH 6.0

A solution of 5.623 g of monobasic sodium phosphate monohydrate, 2.48 g of dibasic sodium phosphate heptahydrate, and 8.500 g of sodium chloride in 800 mL of water for injection was prepared and brought to 1.000 L with water for injection. The pH was checked and if found to fall outside the range of 5.9 to 6.1, the solution was discarded.

Preparation of Stability Samples: PEG-S-mSOD was dialyzed against the buffer of interest for 24 hours at 4°C. The protein concentration was approximately 3.3 mg/mL. The solutions were filtered through 4 mm sterile Millex-GV 0.22 µm filters and added directly to glass vials under sterile conditions. Kimble 2 mL or 5 mL USP type I glass vials, West 4416/50 gray Teflon-coated butyl stoppers, and West FO Lacq crimp tops were used. The fill volume was usually 1-3 mL. The samples were stored in the dark at constant temperature. The stability of each sample was determined by monitoring its enzyme activity, the amount of free PEG, and its solution clarity. The pH of the solution was also checked to ensure sufficient buffer capacity.

Determination of Free PEG: The concentration of free PEG (both R = H and R = methyl) in the solution was determined by size exclusion HPLC. A TSK 2000 PWXL column and a TSK 3000 PWXL column were connected in a series. The mobile phase consisted of 0.1 M NaCl, 0.1 M NaPᵢ buffer, pH 6.7. The chromatography separated free PEG from PEG-S-mSOD. The PEG-S-mSOD peak was detected by a UV detector set at 230 nm and the free PEG peak by a refractive index detector. The amount of free PEG in the sample was determined by integrating the area of the peak. A standard curve of peak area versus concentration of PEG was constructed by injecting solutions of PEG-5000 of known concentrations.

SOD Activity Assay: The enzyme activity of SOD was measured by the method described by McCord and Fridovich (op. cit.).

The initial range of trial pHs was developed based on data from preformulation studies including circular dichroism (CD) spectroscopy, differential scanning calorimetry (DSC), atomic absorption (AA) spectroscopy, and stability tests under various conditions. The DSC data suggested that the pH range of 5.5 to 7 provided maximal stability for mSOD and PEG-S-m-SOD. The decreasing stability of the enzyme at a pH below 5 can be attributed to loss of copper and zinc ions as shown by data from AA studies. Further corroborating data were obtained from CD studies indicating a partial unfolding of SOD at pHs below 5.0.

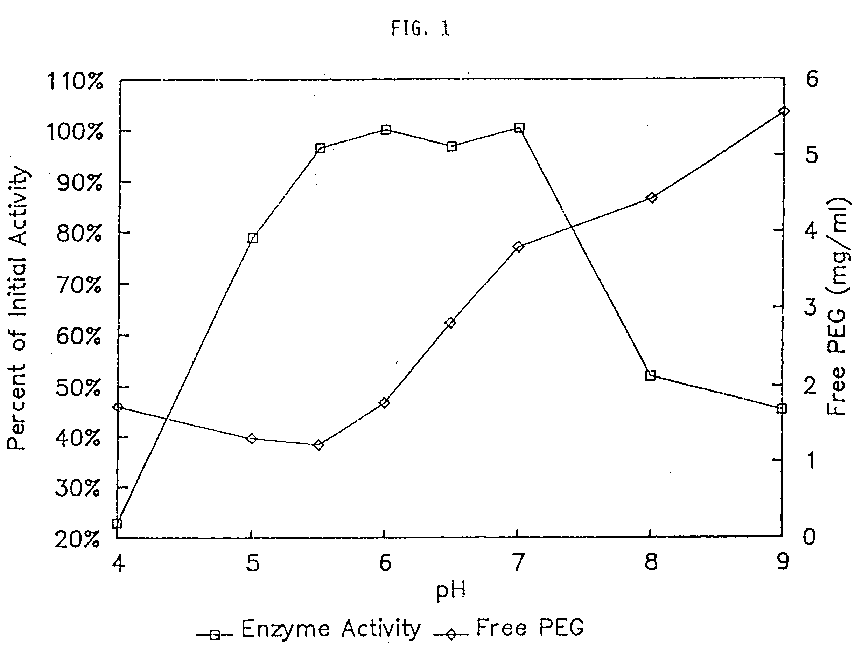

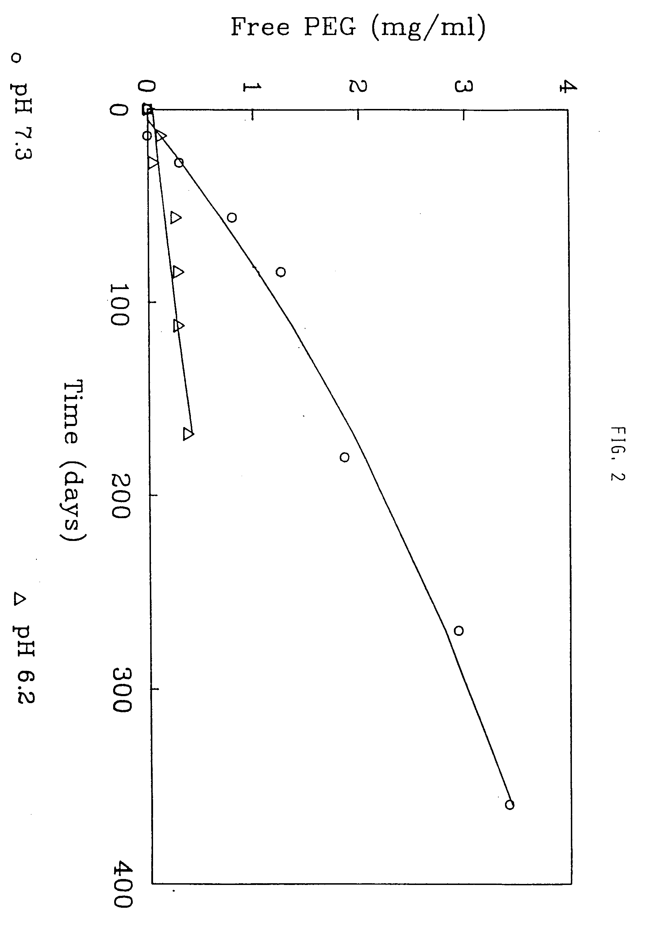

HPLC analysis of free PEG in solution showed that the optimum pH range for stability of the succinyl linker was below 6.5. At pHs above this range, PEG-S-mSOD showed higher rates of release of PEG into the solution due most likely to the hydrolysis of the ester bond to the succinyl group. The results of the initial studies are presented in Figure 1. The results of long-term storage of a solution of example 1 (at pH 6.2) as compared to the formulation of the art (pH 7.3) are shown in Figure 2. The rate of decomposition of PEG-S-mSOD in the solution of the art is about seven-fold higher than its decomposition in a solution of the invention.

## Claims

1. An aqueous formulation of PEG-S-mSOD comprising PEG-S-mSOD and a non-chelating buffer in the range from pH 5.0 to pH 6.5.

2. A formulation according to claim 1 wherein said buffer is present at a concentration from 10 mM to 500 mM.

3. A formulation according to claim 1 wherein said buffer is a phosphate, adipate maleate or glutarate.

4. A formulation according to claim 2 wherein said buffer is a phosphate and said pH is from 6.0 to 6.2.

5. A formulation according to claim 2 wherein said buffer is an adipate and said pH is from 5.5 to 5.8.

6. A formulation according to claim 4 which comprises from 20,000 to 30,000 U of PEG-S-mSOD, from 30 to 50 mM phosphate buffer and from 140 to 150 mM sodium chloride per mL.

7. A formulation according to claim 5 which comprises from 20,000 to 30,000 U of PEG-S-mSOD, from 30 to 50 mM adipate buffer and from 140 to 150 mM sodium chloride per mL.

8. A method for treating or preventing oxidative injury in a mammal which comprises administering a formulation according to claim 1.

9. A method for treating or preventing oxidative injury in a mammal which comprises administering a formulation according to claim 6.

10. A method for treating or preventing oxidative injury in a mammal which comprises administering a formulation according to claim 7.
